# EUROPÄISCHE PATENTSCHRIFT

(11) **EP 0 467 164 B1**
(45) Veröffentlichungstag und Bekanntmachung des Hinweises auf die Patenterteilung: **31.01.1996**
(21) Anmeldenummer: 91111124.3
(22) Anmeldetag: 04.07.1991
(51) Int. Cl.: A61K 31/44

(54) **Flupirtin in Kombination mit Antiparkinsonika zur Bekämpfung von Muskelverspannungen**
Flupirtine in combination with antiparkinsonica against muscle bracing
Flupirtine en combinaison avec antiparkinsonica pour lutter contre la raideur musculaire

(30) Priorität: 14.07.1990 DE 4022442
(43) Veröffentlichungstag der Anmeldung: 22.01.1992
(60) Teilanmeldung: 95101189.9
(73) Patentinhaber: ASTA Medica Aktiengesellschaft, D-01277 Dresden (DE)
(72) Erfinder: Lobisch, Michael, Dr., W-6105 Ober-Ramstadt (DE); Venhaus, Ralph, Dr., D-64625 Bensheim (DE); Nickel, Bernd, Dr., W-6109 Mühltal (DE); Szelenyi, Istvan, Prof., W-8501 Schwaig (DE); Engel, Jürgen, Prof., W-8755 Alzenau (DE); Emig, Peter, Dr., W-6369 Niederdorfelden (DE)

(56) Entgegenhaltungen:
- EP-A- 0 189 788
- DE-A- 3 604 575
- ARZNEIMITTEL-FORSCHUNG, Band 35, Nr. 1, 1985, Seiten 44-55; V.V. JAKOVLEV et al.: "Allgemeine pharmakologische Untersuchungen mit dem Analgetikum Flupirtin"
- ARZNEIMITTEL-FORSCHUNG, Band 40(II), Nr. 8, 1990, Seiten 909-911; B. NICKEL et al.: "Einfluss von Flupirtin, verschiedener Analgetika und Muskelrelaxantien auf den Skelettmuskeltonus wacher Ratten"
- FORTSCHR. MED., vol. 109, no. 6, 28. Februar 1991, Seiten 158-160; R. WÖRZ: "Flupirtin bei chronischen myofasziellen Schmerzzuständen"
- PHARMAZEUTISCHE ZEITUNG, Band 136, Nr. 30, 28. Februar 1991, Seite 28; J. BRÜGGMANN: "Flupirtin bei Verspannungen der Muskulatur"
- ALLGEMEINE UND SPEZIELLE PHARMAKOLOGIE UND TOXICOLOGIE, Auflage 5, 1987, Seiten 519-521; "Antiparkinsonmittel", Bi Wissenschaftsverlag, Mannheim, DE
- THE PHARMACOLOGICAL BASIS OF THERAPEUTICS, Goodman and Gilman's, 7th ed., pp.483-484.

## Beschreibung

Der Arzneimittelwirkstoff Flupirtin (chemische Bezeichnung 2-Amino-3-carbethoxyamino-6-(4-fluorbenzylamino)-pyridin ist ein Analgetikum. Die Herstellung dieser Verbindung und deren physiologisch verwendbare Salze sind in der deutschen Patentschrift DE 17 95 858 sowie in DE 31 33 519 beschrieben.

Als Antiparkinsonika werden insbesondere Dopaminergika wie L-Dopa und (-)-Deprenyl und Anticholergika eingesetzt. In dem DE-Patent 36 04 575 wird die Kombination von Flupirtin mit anticholinerg wirksamen Spasmolytika beschrieben, wobei hierbei eine überadditive spasmolytische Wirkung erreicht wird.

Die Aufgabe der Erfindung besteht darin, ein Arzneimittel mit verstärkter skelettmuskelrelaxierender Wirkung, zur Bekämpfung von Muskelverspannungen, insbesondere zur Behandlung von Parkinson-Erkrankungen zur Verfügung zu stellen.

Es wurde nun gefunden, daß Flupirtin in Kombination mit Antiparkinsonika eine hohe skelettmuskelentspannende Wirkungen besitzt und für die Bekämpfung von Muskelverspannungen, zum Beispiel bei der Behandlung von Parkinson-Erkrankungen, geeignet ist.

Die Kombination von Flupirtin mit solchen Antiparkinsonika führt zu einer überadditiv verstärkten skelettmuskel-relaxierenden Wirkung. Flupirtin setzt also beispielsweise den Tonus der Skelettmuskulatur in Erkrankungen, in denen der Muskeltonus erhöht ist, wie z.B. Morbus Parkinson, herab.

Die muskelentspannende Wirkung des Flupirtins führt auch zu einer Verminderung der ähnlich wie bei den klassischen Antiparkinsonika durch Resperpin induzierten Rigidität.

### Versuchsmethode

Als Versuchstiere wurden weibliche SIV 50-Ratten, 160 g bis 200 g, eingesetzt. Die Tiere wurden bei Raumtemperaturen von 20°C bis 24°C, einer relativen Luftfeuchtigkeit von 60 % bis 70 % und Hell-Dunkel-Zeiten von 12 zu 12 Stunden gehalten. Futter (Atromin®, Altrogge) und Wasser standen adlibitum zur Verfügung.
Flupirtin wurde als Maleat eingesetzt.

Die muskelentspannende Wirkung wurde durch kontinuierliche Messung des Widerstandes des Flexor- und Extensor-Muskels am Hinterbein der Ratten gemessen. Dazu wurde der Fuß des rechten Hinterbeins der Ratte durch einen elektronisch angesteuerten Stellmotor in einem zeitlichen Abstand von 30 Sekunden schnell nach oben und danach nach unten bewegt. Die Reflexreaktion von Flexor und Extensor der Ratten, hervorgerufen durch die zwanghafte Bewegung des Rattenfußes, wurde auf einen Kraftaufnehmer übertragen und rechnergestützt (IBM/PC) ausgewertet. Parallel zur on-line-Auswertung wurden die Daten auf einem hierfür üblichen Schreiber mitgeschrieben und gleichzeitig auf einem Magnetband abgespeichert. Vor Versuchsbeginn wurde die Versuchseinheit durch Auflegen eines geeichten 50g-Gewichtes auf den Kraftaufnehmer kalibriert. Danach wurden die Tiere in die Versuchsanordung gesetzt und hatten 30 Minuten Zeit, sich zu adaptieren. Nach Ermittlung des Ausgangswiderstands von Flexor und Extensor der Ratten (Kontrollwerte) wurde Flupirtin-Maleat intraperitoneal appliziert und der Einfluß der Verbindungen auf Flexor und Extensor über 90 Minuten, wobei eine Mitteilung über jeweils 10 Minuten erfolgte, kontinuierlich registriert, abgespeichert und on-line ausgewertet.

Die Unterschiede zwischen den Mittelwerten der Versuchstiergruppen wurden mit dem t-Test nach Student auf Signifikanz geprüft.

Nach intraperitonealer Gabe von Flupirtin im analgetisch wirksamen Dosenbereich wurde ein muskelentspannender Effekt festgestellt, wobei in dem untersuchten Dosenbereich bei den mit Flupirtin behandelten Tieren keine zentralen Nebenwirkungen wie Ataxie beziehungsweise Verminderung der Spontanmotilität beobachtet wurden.

Beispielsweise wird bei obengenannter Versuchsmethode bei einer Dosis von 1 mg/Körpergewicht kg Ratte die medikamentös induzierte Rigidität der Skelettmuskulatur in der wachen Ratte um 50 % reduziert.

Die niedrigste, bereits wirksame Dosis in dem oben angegebenen Tierversuch ist beispielsweise
1 mg/kg oral
0,1 mg/kg intraperitoneal
Als allgemeiner Dosisbereich für die Wirkung (Tierversuch wie oben) kommt beispielsweise in Frage:
1 - 100 mg/kg oral, insbesondere 10 bis 40 mg/kg
0,1 - 30 mg/kg intraperitoneal, insbesondere 1 bis 20 mg/kg
Zu anderen Wirkstoffen gleicher Wirkungsrichtung zeigt das Flupirtin insbesondere folgende Unterschiede: Flupirtin ruft keine Abhängigkeit hervor und besitzt eine starke analgetische Wirkung.

Die Wirkung von Flupirtin in Kombination mit bekannten Antiparkinson-Mitteln wie z.B. (-)-Deprenyl, Biperiden und L-Dopa auf die reserpininduzierte Rigidität geht aus der folgenden Tabelle hervor.

**Tabelle**

| Rigiditätsabnahme | | | |
|---|---|---|---|
| Substanz | Dosis * mg/kg i.p | Flexor %Wirkung | Extensor %Wirkung |
| Flupirtin | 5 | 53 | 56 |
| (-)-Deprenyl | 1 | 18 | 14 |
| Flupirtin + (-)-Deprenyl | 5 + 1 | 96 | 93 |
| Biperiden | 0,1 | 12 | 16 |
| Flupirtin + Biperiden | 5 + 0,1 | 89 | 96 |
| L-Dopa | 5 | 17 | 11 |
| Flupirtin + L-Dopa | 5 + 5 | 93 | 88 |

| | | | |
|---|---|---|---|
| *i.p.=intraperitoneal | | | |

Diese Rigiditätsabnahme wird durch die bereits beschriebene kontinuierliche Messung des Widerstandes des Flexor- und Extensor-Muskels am Hinterbein der Ratte bestimmt. Der einzige Unterschied bei der Bestimmung der Rigiditätsabnahme besteht darin, daß den Tieren 16 Stunden vor Versuchsbeginn Reserpin in einer Dosis von 2,5 mg/kg Ratte intraperitoneal appliziert wird. Das Reserpin erzeugt die Rigidität. Die Abnahme beziehungsweise Aufhebung dieser Rigidität durch Flupirtin in Kombination mit verschiedenen Antiparkinsonika wird dann wie angegeben bestimmt.

Die pharmazeutischen Zubereitungen enthalten im allgemeinen zwischen 50 mg bis 500 mg, vorzugsweise 100 mg bis 200 mg Flupirtin-Base.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen.

Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Kapseln oder Tabletten, die zwischen 100 mg und 200 mg oder Lösungen, die zwischen 0,1 bis 10 Gewichts% Flupirtin enthalten.

Die Einzeldosis an Flupirtin-Base kann beispielsweise liegen
a) bei oralen Arzneiformen zwischen 70 mg und 300 mg, vorzugsweise 100 mg bis 200 mg,
b) bei parenteralen Arzneiformen (zum Beispiels intravenös, intramuskulär) zwischen 8 mg und 80 mg, vorzugsweise 10 mg und 100 mg.

Die Dosen sind jeweils bezogen auf die freie Flupirtin-Base.

Beispielsweise können 3 mal täglich 1 bis 2 Kapseln oder Tabletten mit einem Gehalt von 50 mg bis 200 mg wirksamer Substanz empfohlen werden.

Das Gewichtsverhältnis von Flupirtin zu Antiparkinsonika liegt zwischen 1 : 0,01 bis 1 : 1.

Die akute Toxizität des Flupirtins an der Maus (ausgedrückt durch die LD 50 mg/kg; Methode nach Miller und Tinter: Proc. Soc. Exper. Biol. a. Med. 57 (1944) 261) liegt beispielsweise bei oraler Applikation zwischen 600 mg/kg und 800 mg/kg (beziehungsweise oberhalb 500 mg/kg.

Die Erfindung betrifft somit eine pharmazeutische synergetische Kombination enthaltend Flupirtin oder dessen therapeutisch verwertbare Salze und Antiparkinsonika, ausgenommen anticholinerge Mittel, zur gleichzeitigen, getrennten oder zeitlich aubgestuften Anwendung zur Bekämpfung von Muskelverspannungen, zum Beispiel bei der Behandlung von Parkinson-Erkrankungen.

Die Erfindung betrifft desweiteren Verfahren zur Herstellung eines Arzneimittels zur Bekämpfung von Muskelverspannungen, zum Beispiel bei der Behandlung von Parkinson-Erkrankungen, welches darin besteht, daß Flupirtin oder dessen therapeutisch verwertbare Salze sowie das Antiparkinsonikum mit üblichen pharmazeutischen Träger-, Hilfs- und/oder Verdünnungsmitteln formuliert werden.

Die Erfindung betrifft desweiteren die Verwendung von Flupirtin oder dessen therapeutisch verwertbaren Salzen und Antiparkinsonika zur Herstellung eines Arzneimittels zur Bekämpfung von Muskelverspannungen, zum Beispiel bei der Behandlung von Parkinson-Erkrankungen.

Flupirtin oder ein pharmazeutisch verwendbares Salz des Flupirtins sowie ein Antiparkinsonikum werden zusammen mit üblichen Träger- und/oder Verdünnungs- beziehungsweise Hilfsstoffen bei Temperaturen zwischen 0 und 120°C, vorzugsweise 20 und 80°C vermischt beziehungsweise homogenisiet und gegebenenfalls die so erhaltene Mischung zur Herstellung von Zubereitungen, die in der Dosierungseinheit 50 bis 500 mg Flupirtin oder ein pharmazeutisch verwendbares Salz des Flupirtins enthalten, in Hohlzellen entsprechender Größe abgefüllt oder granuliert und dann gegebenenfalls unter Zusatz von weiteren üblichen Hilfsstoffen zu Tabletten verpreßt oder in Kapseln abgefüllt.

Flupirtin oder ein pharmazeutisch verwendbares Salz des Flupirtins in Kombination mit einem Antiparkinsonikum, kann mit einem oder mehreren der folgenden Stoffe: Stärke, Cyclodextrin, Harnstoff, Cellulose, Lactose, Formalin-Casein, modifizierte Stärke, Magnesiumstearat, Calciumhydrogenphosphat, Kieselsäure, Talkum, Phenoxyethanol vermischt, die erhaltene Mischung gegebenenfalls mit einer wässrigen Lösung, die als Bestandteil mindestens Gelatine, Stärke, Polyvinylpyrrolidon, Vinylpyrrolidon-Vinylacetat-Copolymerisat und/oder Polyoxyethylen-sorbitanmonooleat enthält, granuliert, das Granulat gegebenenfalls mit einem oder mehreren der oben genannten Hilfsstoffe homogenisiert, und diese Mischung zu Tabletten verpreßt oder in Kapseln abgefüllt werden.

Desweiteren können ein oder mehrere der folgenden Hilfsstoffe: Sojalecitin, Oxynex, Phenoxyethanol bei Temperaturen zwischen 31 und 65°C in geschmolzenem Hartfett oder anderen, Fettsäureglyceride enthaltenen Mischungen suspendiert und homogenisiert, und anschließend die Mischung in Hohlzellen ausgegossen oder in Kapseln abgefüllt werden.

Flupirtin oder ein pharmazeutisch verwendbares Salz des Flupirtins sowie ein Antiparkinsonikum können bei einer Temperatur zwischen 20 bis 120°C, gegebenenfalls in Gegenwart eines oder mehrerer Emulatoren und/oder Komplexbildnern mit mindestens einem der folgenden Stoffe homogenisiert und/oder emulgiert werden: Wasser, Glycerin, Paraffin, Vaseline, alipatischer Alkohol mit 12 bis 25 C-Atomen, alipatische Monocarbonsäure mit 15 bis 20 C-Atomen, Sormitanmonopalmitat, Polyoxyethylenpolyolfettsäureester, ein- oder mehrwertiger niedrigmolekularer alipatischer Alkohol, Fettsäureglycerid, Wachs, Silikon, Polyethylenglykol, Siliziumdioxid.

Desweiteren können Flupirtin oder ein pharmazeutisch verwendbares Salz des Flupirtins und ein Antiparkinsonikum gegebenenfalls in Anwesenheit eines Komplexbildners und/oder eines Emulgators, bei Temperaturen zwischen 30 und 100°C in Wasser, physiologisch unbedenklichen Alkoholen, Polyglykolen, Polyglykolderivaten, Dimethylsulfoxid,

Triglyceriden, Partialestern des Glycerins, Paraffinen oder Ölen oder Mischungen hiervon auflöst, und gegebenenfalls die erhaltene Lösung mit soviel der genannten Lösungsmittel aufgefüllt werden, daß die Endlösung, Endsuspension oder Endmulsion 0,1 - 10 Gewichtsprozent an Wirkstoff Flupirtin enthält.

Die pharmazeutischen Zusammensetzungen enthalten als Wirkstoff Flupirtin oder seine physiologisch verträglichen Salze. Der Wirkstoff liegt in Kombination mit einem Antiparkinsonikum vor.

Die Kombination von Flupirtin mit einem Antiparkinsonika kann gleichzeitig, getrennt oder zeitlich abgestuft verabreicht werden.

Als weitere Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angeben sind:
Ullmanns Encyklopädie der technischen Chemie, Band 4 (1953), Seite 1 bis 39;
Journal of Pharmaceutical Sciences, Band 52 (1963), Seite 918 und ff.;
H.v.Czetsch-Lindenwald, Hilfsstoffe für Pharmazie und angrenzende Gebiete;
Pharm. Ind., Heft 2 (1961), Seite 72 und ff.;
Dr. H.P. Fiedler, Lexikon der Hilfsstoffe für Pharmazie, Kosmetik und angrenzende Gebiete Cantor KG. Aulendorf in Württemberg 1981.

Beispiele hierfür sind Gelatine, natürliche Zucker wie Rohrzucker oder Milchzucker, Lecithin, Pektin, Stärke (zum Beispiel Maisstärke), Cyclodextrine und Cyclodextrinderivate, Polyvinylpyrrolidon, Polyvinylacetat, Gelatine, Gummi arabicum, Alginsäure, Tylose, Talkum Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropylmethylcellulose, Hydroxypropylmethylcellulosephthalat); Fettsäuren sowie Magnesium-, Calcium- oder Aluminiumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnußöl, Rizinusöl, Olivenöl, Sesamöl, Baumwollsaatöl, Maisöl, Weizenkeimöl, Sonnenblumensamenöl, Kabeljau-Leberöl, jeweils auch hydriert; Mono-, Di- und Triglyceride von gesättigten Fettsäuren C₁₂H₂₄O₂ bis C₁₈H₃₆O₂ und deren Gemische, pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 - 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen, Essigsäure, Benzylbenzoat, Dioxolane, Glyzerinformale, Tetrahydrofurfurylalkohol, Polyglykolether mit C₁-C₁₂-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden.

Als solche kommen beispielsweise in Frage: Polymerisate sowie Copolymerisate der Acrylsäure und/oder Methacrylsäure und/oder deren Ester; Copolymerisate aus Acryl- und Methacrylsäureestern mit einem geringen Gehalt an Ammoniumgruppen (zum Beispiel Eudragit® RS), Copolymerisate aus Acryl- und Methacrylsäureestern und Trimethylammoniummethacrylat (zum Beispiel Eudragit®RL); Polyvinylacetat; Fette, Öle, Wachse, Fettalkohole; Hydroxypropylmethylcellulosephthalat oder -acetatsuccinat; Cellulose-, Stärke- sowie Polyvinylacetatphthalat; Carboxymethylcellulose; Methylcellulose-phthalat, - succinat, -phthalatsuccinat sowie -phthalatsäurehalbester; Zein; Ethylcellulose sowie -succinat; Schellack, Gluten; Ethylcarboxyethylcellulose; Ethacrylat-Maleinsäureanhydrid-Copolymer; Maleinsäureanhydrid-Vinylmethylether-Copolymer; Styrol-Maleinsäure-Copolymerisate; 2-Ethyl-hexyl-acrylatmaleinsäureanhydrid; Crotonsäure-Vinylacetat-Copolymer; Glutaminsäure/Glutaminsäureester-Copolymer; Acarboxymethylethyl-celluloseglycerinmonooctanoat; Celluloseacetatsuccinat; Polyarginin.

Als Plastifizierungsmittel für Hüllstoffe können in Frage kommen:
Citronen- und Weinsäureester (Acetyltriethyl-, Acetyltributyl-, Tributyl-, Triethyl-citrat); Glycerin und Glycerinester (Glycerindiacetat, -triacetat, acetylierte Monoglyceride, Rizinusöl); Phthalsäureester (Dibutyl-, Diamyl-, Diethyl-, Dimethyl-, Dipropyl-phthalat), D-(2-Methoxy- oder ethoxyethyl)-phthalat, Ethylphthalyl-, Butylphthalylethyl- und Butylglycolat; Alkohole (Propylenglycol, Polyethylenglycol verschiedener Kettenlängen), Adipate (Di-ethyl-adipat, Di(2-Methoxy- oder ethoxyethyladipat); Benzophenon; Diethyl- und
Dibutylsebacat, -succinat, -tartrat; Diethylenglycoldipropionat; Ethylenglykoldiacetat, - dibutyrat, -dipropionat; Tributylphosphat, Tributyrin; Polyethylenglykolsorbitanmonooleat (Polysorbate wie Polysorbat 80); Sorbitanmooleat.

Zur Herstellung von Lösungen oder Suspensionen kommen beispielsweise Wasser oder physiologisch verträgliche organische Lösungsmittel in Frage, wie zum Beispiel Ethanol, Propanol, Isopropanol, 1,2-Propylenglykol, Polyglykole und deren Derivate, Dimethylsulfoxid, Fettalkohole, Triglyceride, Partialester des Glycerins, Paraffine und ähnliche.
Für injizierbare Lösungen oder Suspensionen kommen zum Beispiel nicht-toxische parenteral verträgliche Verdünnungsmittel oder Lösungsmittel in Frage, wie zum Beispiel: Wasser, 1,3-Butandiol, Ethanol, 1,2-Propylenglykol, Polyglykole in Mischung mit Wasser, Ringer's Lösung, isotonische Kochsalzlösung oder auch gehärtete Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage:
Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fett, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)-imidazolidon-(2).
Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.
Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solche, die Ölsäurereste enthalten) mit Ethylenoxyd erhalten werden (zum Beispiel 40 Mol Ethylenoxy pro Mol Glycerid).
Beispiele für Oleotriglyceride sind Olivenöl, Erdnußöl, Rizinusöl, Sesamöl, Baumwollsaatöl, Maisöl. Siehe auch Dr. H.P. Fiedler "Lexikon der Hilfstoffe für Pharmazie, Kosmetik und angrenzende Gebiete" S. 191-195.

Darüber hinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Süßmitteln, Farbstoffen, Antioxydantien und Komplexbildnern (zum Beispiel Ethylendiaminotetraessigsäure) und dergleichen möglich. Gegebenenfalls ist zur Stabilisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Zur Herstellung von dermal anzuwendenden Zubereitungen kommen in Frage die vorgenannten Substanzen und streichfähige oder flüssige Kohlenwasserstoffe wie Vaselin oder Paraffin oder Gele aus Paraffinkohlenwasserstoffen und Polyethylen, Fette und Öle pflanzlichen oder tierischen Ursprungs, die zum Teil auch hydriert sein können oder synthetische Fette wie Glyceride der Fettsäuren C₈-C₁₈, sodann Bienenwachs, Cetylpalmitat, Wollwachs, Wollwachsalkohole; Fettalkohole wie Cetylalkohol, Stearylalkohol, Polyethylenglykole vom Molekulargewicht 200 bis 20 000; flüssige Wachse wie Isopropylmyristat, Isopropylstearat, Äthyloleat; Emulgatoren wie Natrium-,

Kalium-, Ammoniumsalze der Stearinsäure oder Palmitinsäure sowie Triethanolaminstearat, Alkalisalze der Ölsäure, Ricinolsäure, Salze von sulfurierten Fettalkoholen wie Natriumlaurylsulfat, Natriumcetylsulfat, Natriumstearylsulfat, Salze der Gallensäure, Sterole wie Cholesterol, Partialfettsäureester mehrwertiger Alkohole wie Ethylenglykolmonostearat, Partialfettsäureester des Sorbitans, Partialfettsäureester des Polyoxyethylensorbitans, Sorbitolether des Polyoxyethylens, Fettsäureester des Polyoxyethylens, Fettalkoholether des Polyoxyethylens, Fettsäureester der Saccharose, Fettsäureester des Polyglycerols, Lecithin.

Als Antioxydantien kommen beispielsweise Natriummetabisulfit, Ascorbinsäure, Gallussäure, Gallussäurealkylester, Butylhydroxyanisol, Nordihydroguajaretsäure, Tocopherole sowie Tocopherole + Synergisten (Stoffe, die Schermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich. Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der erfindungsgemäßen Verbindungen erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80°C, vorzugsweise 20 bis 50°C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation kann auf die Haut oder Schleimhaut oder in das Körperinnere erfolgen, beispielsweise oral, enteral, pulmonal, rectal, nasal, vaginal, lingual, intravenös, intraarteriell, intrakardial, intramuskulär, intraperitoneal, intracutan, subcutan. Bei den parenteralen Zubereitungsformen handelt es sich insbesondere um sterile beziehungsweise sterilisierte Erzeugnisse.

## Patentansprüche (Patentansprüche für folgende(n) Vertragsstaat(en): AT, BE, CH, DE, DK, FR, GB, IT, LI, LU, NL, SE)

1. Pharmazeutische synergetische Kombination enthaltend Flupirtin oder dessen therapeutisch verwertbare Salze und Antiparkinsonika , ausgenommen anticholinerge Mittel, zur gleichzeitigen, getrennten oder zeitlich abgestuften Anwendung zur Bekämpfung von Muskelverspannungen, zum Beispiel bei der Behandlung von Parkinson-Erkrankungen.

2. Pharmazeutische Kombination gemäß Anspruch 1, dadurch gekennzeichnet, daß als Antiparkinsonika (-)-Deprenyl oder L-Dopa enthalten sind.

3. Mittel nach Anspruch 1, dadurch gekennzeichnet, daß es übliche pharmazeutische Träger-, Hilfs- und/oder Verdünnungsmittel enthält.

4. Verfahren zur Herstellung eines Arzneimittels nach Anspruch 1 zur Bekämpfung von Muskelverspannungen, zum Beispeil bei der Behandlung von Parkinson-Erkrankungen dadurch gekennzeichnet, daß Flupirtin oder dessen therapeutisch verwertbare Salze, und die Antiparkinsonika mit üblichen pharmazeutischen Träger-, Hilfs- und/oder Verdünnungsmitteln formuliert wird.

5. Verfahren nach Anspruch 4 dadurch gekennzeichnet, daß man Flupirtin oder ein pharmazeutisch verwendbares Salz des Flupirtins und die Antiparkinsonika, zusammen mit üblichen Träger- und/oder Verdünnungs- beziehungsweise Hilfsstoffen bei Temperaturen zwischen 0 und 120°C, vorzugsweise 20 und 80°C vermischt beziehungsweise homogenisiert und gegebenenfalls die so erhaltene Mischung in Hohlzellen entsprechender Größe abfüllt oder granuliert und dann gegebenenfalls unter Zusatz von weiteren üblichen Hilfsstoffen zu Tabletten verpreßt oder in Kapseln abfüllt.

6. Verfahren nach Anspruch 4 dadurch gekennzeichnet, daß man Flupirtin oder ein pharmazeutisch verwendbares Salz des Flupirtins und die Antiparkinsonika, gegebenenfalls in Anwesenheit eines Komplexbildners und/oder eines Emulgators, bei Temperaturen zwischen 30 und 100°C in Wasser, physiologisch unbedenklichen Alkoholen, Polyglykolen, Polyglykolderivaten, Dimethylsulfoxid, Triglyceriden, Partialestern des Glycerins, Paraffinen oder Ölen oder Mischungen hiervon auflöst, und gegebenenfalls die erhaltene Lösung mit den genannten Lösungsmittel auffüllt um die gewünschte Endlösung, Endsuspension oder Endemulsion zu erhalten.

7. Verwendung von Flupirtin oder einem pharmazeutisch verwendbarem Salz des Flupirtins und Antiparkinsonika zur Herstellung eines Arzneimittels zur Bekämpfung von Muskelverspannungen, zum Beispeil bei der Behandlung von Parkinson-Erkrankungen.

## Patentansprüche (Patentansprüche für folgende(n) Vertragsstaat(en): ES, GR)

1. Verfahren zur Herstellung eines Arzneimittels zur Bekämpfung von Muskelverspannungen, zum Beispiel bei der Behandlung von Parkinson-Erkrankungen dadurch gekennzeichnet, daß Flupirtin oder dessen therapeutisch verwertbare Salze, und die Antiparkinsonika mit üblichen pharmazeutischen Träger-, Hilfs- und/oder Verdünnungsmitteln formuliert wird.

2. Verfahren nach Anspruch 1 dadurch gekennzeichnet, daß man Flupirtin oder ein pharmazeutisch verwendbares Salz des Flupirtins und die Antiparkinsonika, zusammen mit üblichen Träger- und/oder Verdünnungs- beziehungsweise Hilfsstoffen bei Temperaturen zwischen 0 und 120°C, vorzugsweise 20 und 80°C vermischt beziehungsweise homogenisiert und gegebenenfalls die so erhaltene Mischung in Hohlzellen entsprechender Größe abfüllt oder granuliert und dann gegebenenfalls unter Zusatz von weiteren üblichen Hilfsstoffen zu Tabletten verpreßt oder in Kapseln abfüllt.

3. Verfahren nach Anspruch 1 dadurch gekennzeichnet, daß man Flupirtin oder ein pharmazeutisch verwendbares Salz des Flupirtins und die Antiparkinsonika, gegebenenfalls in Anwesenheit eines Komplexbildners und/oder eines Emulgators, bei Temperaturen zwischen 30 und 100°C in Wasser, physiologisch unbedenklichen Alkoholen, Polyglykolen, Polyglykolderivaten, Dimethylsulfoxid, Triglyceriden, Partialestern des Glycerins, Paraffinen oder Ölen oder Mischungen hiervon auflöst, und gegebenenfalls die erhaltene Lösung mit den genannten Lösungsmittel auffüllt um die gewünschte Endlösung, Endsuspension oder Endemulsion zu erhalten.

4. Verwendung von Flupirtin oder einem pharmazeutisch verwendbarem Salz des Flupirtins und Antiparkinsonika zur Herstellung eines Arzneimittels zur Bekämpfung von Muskelverspannungen, zum Beispiel bei der Behandlung von Parkinson-Erkrankungen.

## Claims (Claims for the following Contracting State(s): AT, BE, CH, DE, DK, FR, GB, IT, LI, LU, NL, SE)

1. Pharmaceutical, synergistic combination containing flupirtine or the therapeutically acceptable salts thereof and an antiparkinsonian, with the exception of anticholinergic agents, for simultaneous, separate or time-spaced use for combatting muscle spasm, for example in the treatment of Parkinsonian disorders.

2. Pharmaceutical combination according to claim 1, characterised in that it contains (-)-deprenyl or L-dopa as the antiparkinsonian.

3. Agent according to claim 1, characterised in that it contains conventional pharmaceutical carriers, auxiliary agents and/or diluents.

4. Process for the production of a pharmaceutical preparation according to claim 1 for combatting muscular spasm, for example in the treatment of Parkinsonian disorders, characterised in that flupirtine or the therapeutically acceptable salts thereof and the antiparkinsonian are formulated with conventional pharmaceutical carriers, auxiliary substances and/or diluents.

5. Process according to claim 4, characterised in that flupirtine or a pharmaceutically acceptable salt of flupirtine and the antiparkinsonian, together with conventional carriers and/or diluents or auxiliary substances, are mixed or homogenised at temperatures of between 0 and 120°C, preferably 20 and 80°C and the mixture so obtained is optionally poured into hollow cells of an appropriate size or pelletised and then, optionally with the addition of further conventional auxiliary substances, pressed into tablets or enclosed in capsules.

6. Process according to claim 4, characterised in that flupirtine or a pharmaceutically acceptable salt of flupirtine and the antiparkinsonian are dissolved, optionally in the presence of a complexing agent and/or an emulsifier, at temperatures of between 30 and 100°C in water, physiologically harmless alcohols, polyglycols, polyglycol derivatives, dimethyl sulphoxide, triglycerides, partial esters of glycerol, paraffins or oils or mixtures thereof and the resultant solution is optionally made up with the stated solvents in order to obtain the desired final solution, final suspension or final emulsion.

7. Use of flupirtine or a pharmaceutically acceptable salt of flupirtine and an antiparkinsonian for the production of a pharmaceutical preparation for combatting muscle spasm, for example in the treatment of Parkinsonian conditions.

## Claims (Claims for the following Contracting State(s): ES, GR)

1. Process for the production of a pharmaceutical preparation for combatting muscular spasm, for example in the treatment of Parkinsonian disorders, characterised in that flupirtine or the therapeutically acceptable salts thereof and the antiparkinsonian are formulated with conventional pharmaceutical carriers, auxiliary substances and/or diluents.

2. Process according to claim 1, characterised in that flupirtine or a pharmaceutically acceptable salt of flupirtine and the antiparkinsonian, together with conventional carriers and/or diluents or auxiliary substances, are mixed or homogenised at temperatures of between 0 and 120°C, preferably 20 and 80°C and the mixture so obtained is optionally poured into hollow cells of an appropriate size or pelletised and then, optionally with the addition of further conventional auxiliary substances, pressed into tablets or enclosed in capsules.

3. Process according to claim 1, characterised in that flupirtine or a pharmaceutically acceptable salt of flupirtine and the antiparkinsonian are dissolved, optionally in the presence of a complexing agent and/or an emulsifier, at temperatures of between 30 and 100°C in water, physiologically harmless alcohols, polyglycols, polyglycol derivatives, dimethyl sulphoxide, triglycerides, partial esters of glycerol, paraffins or oils or mixtures thereof and the resultant solution is optionally made up with the stated solvents in order to obtain the desired final solution, final suspension of final emulsion.

4. Use of flupirtine or a pharmaceutically acceptable salt of flupirtine and an antiparkinsonian for the production of a pharmaceutical preparation for combatting muscle spasm, for example in the treatment of Parkinsonian conditions.

## Revendications (Revendications pour l'(les) Etat(s) contractant(s) suivant(s): AT, BE, CH, DE, DK, FR, GB, IT, LI, LU, NL, SE)

1. Combinaison pharmaceutique synergique contenant de la Flupirtine ou ses sels thérapeutiquement utilisables et des agents antiparkinsoniens, sauf les agents antichlolinergiques, pour une utilisation simultanée, séparée ou étagée dans le temps pour combattre les contractures musculaires, par exemple lors du traitement des maladies de Parkinson.

2. Combinaison pharmaceutique selon la revendication 1, caractérisée en ce qu'elle contient comme agents antiparkinsoniens (-)-déprényl et L-dopa.

3. Moyen selon la revendication 1, caractérisé en ce qu'il contient les véhicules, additifs et/ou diluants pharmaceutiques habituels.

4. Procédé de production d'un médicament selon la revendication 1 pour combattre les contractures musculaires, par exemple dans le traitement des maladies de Parkinson, caractérisé en ce qu'on formule la Flupirtine ou ses sels thérapeutiquement utilisables et les agents antiparkinsoniens avec les véhicules, additifs et/ou diluants pharmaceutiques usuels.

5. Procédé selon la revendication 4, caractérisé en ce qu'on mélange la Flupirtine ou un sel de la Flupirtine pharmaceutiquement utilisable et les agents antiparkinsoniens aux véhicules et/ou diluants additifs à des températures comprises entre 0 et 120°C, de préférence entre 20 et 80°C ou on homogénéise et le cas échéant on conditionne le mélange ainsi obtenu dans des cellules vides de taille appropriée ou on granule et après addition le cas échéant d'autres additifs usuels on comprime en comprimés ou on conditionne dans des gelules.

6. Procédé selon la revendication 4, caractérisé en ce qu'on dissout la Flupirtine ou un sel de Flupirtine pharmaceutiquement utilisable et les agents antiparkinsoniens, le cas échéant en présence d'un complexant et/ou d'un émulsionnant, à des températures comprises entre 30 et 100°C dans l'eau, des alcools physiologiquement non dangereux, des polyglycols, des dérivés de polyglycol, du diméthylsulfoxyde, des triglycérides, des esters partiels de glycérine, des paraffines ou des huiles ou leurs mélanges, et le cas échéant on dilue la solution obtenue avec le solvant cité pour obtenir la solution finale, la suspension finale ou l'émulsion finale souhaitée.

7. Utilisation de Flupirtine ou d'un sel de Flupirtine pharmaceutiquement utilisable et des agents antiparkinsoniens pour produire un médicament pour combattre les contractures musculaires par exemple dans le traitement des maladies de Parkinson.

## Revendications (Revendications pour l'(les) Etat(s) contractant(s) suivant(s): ES, GR)

1. Procédé de production d'un médicament pour combattre les contractures musculaires, par exemple dans le traitement des maladies de Parkinson, caractérisé en ce qu'on formule la Flupirtine ou ses sels thérapeutiquement utilisables et les agents antiparkinsoniens avec les véhicules, additifs et/ou diluants pharmaceutiques usuels.

2. Procédé selon la revendication 1, caractérisé en ce qu'on mélange la Flupirtine ou un sel de la Flupirtine pharmaceutiquement utilisable et les agents antiparkinsoniens aux véhicules et/ou diluants additifs à des températures comprises entre 0 et 120°C, de préférence entre 20 et 80°C ou on homogénéise et le cas échéant on conditionne le mélange ainsi obtenu dans des cellules vides de taille appropriée ou on granule et aprés addition le cas échéant d'autres additifs usuels on comprime en comprimés ou on conditionne dans des gelules.

3. Procédé selon la revendication 1, caractérisé en ce qu'on dissout la Flupirtine ou un sel de Flupirtine pharmaceutiquement utilisable et les agents antiparkinsoniens, le cas échéant en présence d'un complexant et/ou d'un émulsionnant, à des températures comprises entre 30 et 100°C dans l'eau, des alcools physiologiquement non dangereux, des polyglycols, des dérivés de polyglycol, du diméthylsulfoxyde, des triglycérides, des esters partiels de glycérine, des paraffines ou des huiles ou leurs mélanges, et le cas échéant on dilue la solution obtenue avec le solvant cité pour obtenir la solution finale, la suspension finale ou l'émulsion finale souhaitée. finale, la suspension finale ou l'émulsion finale souhaitée.

4. Utilisation de Flupirtine ou d'un sel de Flupirtine pharmaceutiquement utilisable et des agents antiparkinsoniens pour produire un médicament pour combattre les contractures musculaires par exemple dans le traitement des maladies de Parkinson.
